# EUROPEAN PATENT SPECIFICATION

(11) **EP 3 445 389 B1**
(45) Date of publication and mention of the grant of the patent: **26.10.2022**
(21) Application number: 17785561.6
(22) Date of filing: 30.01.2017
(51) Int. Cl.: A61L 26/00, A61K 38/48, A61K 47/00, A61P 17/00, C12N 9/64

(54) **FORMULATIONS FOR DEBRIDEMENT OF CHRONIC WOUNDS**
ZUBEREITUNGEN ZUM DEBRIDEMENT VON CHRONISCHEN WUNDEN
FORMULATIONS POUR LE DÉBRIDEMENT DES PLAIES CHRONIQUES

(30) Priority: 18.04.2016 US 201662323812 P
(43) Date of publication of application: 27.02.2019
(73) Proprietor: Mediwound Ltd, 8122745 Yavne (IL)
(72) Inventor: LOZINSKY, Evgenia, 8465138 Beer Sheva (IL); GEBLINGER, Dafna, 7608654 Rehovot (IL); BARTFELD, Deborah Hanah, 7680400 Mazkeret-Batya (IL); ASCULAI, Eilon, 8533800 Lehavim (IL)
(74) Representative: Straus, Alexander
(86) International application number: PCT/IL2017/050110
(87) International publication number: WO 2017/183018

(56) References cited:
- WO-A1-81/01242
- WO-A1-2010/038231
- WO-A2-2006/054309
- CN-A- 105 213 107
- US-A- 5 575 987
- US-A1- 2009 010 910

## Description

The present invention relates to wound debridement. Particularly, the present invention relates to debridement of chronic wounds comprising topically applying to a wound site a debriding formulation comprising a proteolytic enzyme mixture obtained from bromelain and ananain and a water-soluble gelling agent, the debriding formulation being applied to the wound site up to ten times over a period of up to four weeks, thereby achieving debridement of chronic wounds.

### BACKGROUND OF THE INVENTION

Chronic or hard to heal wounds are a common ailment, afflicting millions of people annually. The majority of chronic wounds are caused by a local or generalized vascular insufficiency that reduces blood flow to the skin and subcutaneous tissue. The most common type of chronic or hard to heal wounds include: pressure ulcers (decubiti or "bed sores"), diabetic ulcers, arterial ulcers; venous ulcers, and post surgical/post trauma ulcers or a combination of these.

Chronic wounds result in a severe damage to the skin. This damage may involve the entire thickness of the skin and may often include deeper tissues. The damaged skin loses the anatomic organization of a healthy skin, the stratum corneum is at least partially destroyed and consequently the inner layers of the skin are no longer protected from the external environment. Moreover, the damaged skin typically contains eschar, diseased and/or abnormal cells that must be removed in order to enable healing. Leaving the eschar in place extends and deepens the damage into the neighboring, undamaged tissues. This eschar also serves as a medium for bacteria growth and a source of infection, contamination and sepsis which may be life threatening.

Studies conducted to investigate the composition and structure of the eschar and necrotic tissue in chronic wounds revealed that it is comprised of multiple protein species representing the extracellular matrix proteins and degradation products of autolytic debridement. These studies indicated that chronic wound debridement is likely to require multiple enzyme specificities to degrade the various constituents of the non viable and necrotic tissues in chronic wounds.

Removal of the eschar, diseased and/or abnormal cells, also known as "debridement", is performed by surgical procedures, by mechanical means (dressings changes, bathing), by autolytic procedures (dressings that promote maceration) or by enzymatic means. Surgery is one of the most common procedures of debridement wherein small necrotic areas are excised of the entire damaged skin. This method is limited to small non-tangential surfaces. It also involves the removal of large fractions of healthy tissue which, if preserved, could serve as a source for the natural healing processes. Surgical procedures are also more expensive and require medical resources.

Enzymatic debridement is advantageous over mechanical and surgical debridement mainly since it is less painful, more selective and does not require the assistance of well-trained medical personnel. The application of proteolytic enzymes for debridement is well known in the art. These enzymes include those isolated from bacteria and those generally found in plant sources, such as papaya (papain), fig (ficin), and pineapple (bromelain). Hydrolytic enzymes derived from the pineapple plant that are useful for digestion, dissection and separation of non-viable, especially eschar tissue, from viable tissue in a mammalian host are described in U.S. Patent Nos. 4,197,291; 4,226,854; 4,307,081; 4,329,430 and 5,830,739, among others.

The degree of the therapeutic activity obtained from topical application of proteolytic enzymes is governed, inter alia, by the intrinsic catalytic characteristics of the enzymes. The major problems associated with topical use of compositions comprising proteolytic enzymes are that the catalytic activity of the enzymes is rapidly attenuated due to the typical low pH at the lesion area, adsorption of the enzyme molecules to the surface of the wound bed and/or the surface of the dressing, and inhibition of enzymatic activity by moieties within the wound exudates. Therefore, obtaining stable enzymatic formulations is complicated.

Several ointments are currently being marketed for debriding eschar, such as Santyl^{®} ointment. These ointments are typically applied daily for several months to achieve the desired wound debridement.

U.S. Pat. No. 4,668,228 to Bolton et al., discloses debriding tapes which contain a proteolytic enzyme useful for debridement of eschar and necrotic tissue, e.g., subtilisin, bromelain, in dry powdered form on the adhesive mass surface of an occlusive or semi-occlusive surgical adhesive tape. According to U.S. Pat. No. 4,668,228, when the debriding tape is applied to a burned surface, water from the wound which cannot penetrate the occlusive tape backing activates the debriding enzymes.

U.S. Pat. No. 4,784,653 to Bolton et al., discloses an absorbent adhesive dressing for use in treating wounds of the ulcer and burn type which comprises a three layer sandwich-type constructions having an occlusive film as the outer layer, an absorbent layer of fibers as the middle layer, and a wet-stick adhesive as the inner wound facing adhesive layer which is made of an acrylic polymer having both hydrophilic and hydrophobic characteristics. According to U.S. Pat. No. 4,784,653, a debriding enzyme may be added to the adhesive mass, if desired.

U.S. Pat. No. 5,271,943 to Bogart et al., discloses therapeutic gels which have a minimum yield point of about 800 poise and a maximum apparent viscosity of about 100,000 cps, which gels comprise water, sodium chloride, and a gelling agent.

U.S. Pat. No. 5,514,370 to Stern et al., discloses pharmaceutical compositions for topical application containing high concentrations of collagenase in non-aqueous excipients. U.S. Pat. No. 5,514,370 further discloses a method of treating a wound which comprises applying thereto a composition consisting essentially of a non-aqueous excipient and collagenase.

U.S. Pat. No. 5,804,213 to Rolf discloses a prepackaged wound dressing which comprises a natural or synthetic hydrocolloid in dry particulate form. According to U.S. Pat. No. 5,804,213, the hydrocolloid in dry particulate form is contained in a compartment of a sealed container separate from moisture. After mixing with water, the admixture is sufficiently fluid to allow it to be poured or spread into a wound. Following application to the wound, the hydrated hydrocolloidal dispersion begins to solidify to form a solid, self-supporting flexible dressing which consists of water, hydrocolloid and a biologically active constituent.

U.S. Pat. No. 6,548,556 to Hobson et al. discloses an enzymatic anhydrous hydrophilic debrider that uses in combination a proteolytic enzyme and an anhydrous hydrophilic Poloxamer carrier.

U.S. Pat. No. 8,062,661 to Caldwell et al. discloses methods of debriding a skin wound which include contacting the skin wound with a hydrogel patch debridement composition and removing the hydrogel patch debridement composition from said skin wound to remove foreign matter from the skin wound.

International Application Publication No. WO 2006/054309 assigned to the applicant of the present invention discloses a debriding composition obtained from bromelain useful in debriding eschar tissues and in wound healing.

International Application Publication No. WO 2013/011514 assigned to the applicant of the present invention discloses a proteolytic extract obtained from bromelain for the treatment of connective tissue diseases which are associated with excess of collagen deposition, including Dupuytren's disease and Peyronie's disease.

U.S. Provisional Patent Application No. 62/289,246 to the applicant of the present invention, filed on January 31, 2016, discloses debriding compositions comprising a proteolytic enzyme mixture obtained from bromelain being in a dry form, and an aqueous gel carrier, wherein, prior to use, the proteolytic enzyme mixture being admixed with the aqueous gel carrier to form a debriding composition useful for debridement and treatment of chronic wounds.

WO8101242 A1 discloses a process for isolating an enzyme mixture for the treatment of devitalized tissue from crude bromelain, comprising the steps of suspending the crude bromelain in a weakly basic buffer, to selectively dissolve the active enzyme mixture; separating the undissolved solids from the solution; and removing small molecules having a molecular weight of 10,000 or less from the solution. The enzyme mixture is dissolved in an inert solvent carrier containing at least 60 percent water prior to application to the devitalized tissue.

CN105213107 A inter alia discloses a method to provide a bromelain gel dressing comprising adding bromelain to gel substrate according to a percentage of 0.05% to 5%; then evenly stirring the mixture for 1 to 100mins; canning and disinfecting the mixture to become sterile.

There is a long-felt and unmet need for improved enzymatic debridement of chronic wounds which achieve complete wound debridement and facilitate closure and healing of chronic wounds.

### SUMMARY OF THE INVENTION

The present invention provides a debridement formulation for use in the debridement of wounds, particularly of chronic wounds, comprising topically applying to a wound site a debriding formulation in a regimen of up to ten applications during a time period of up to four weeks, wherein the debriding formulation is formulated as a hydrogel comprising: (a) a composition in a dry or powdered form comprising (i) a proteolytic enzyme mixture obtained from bromelain comprising stem bromelain (EC 3.4.22.32) and ananain (EC 3.4.22.31); and (ii) a water-soluble gelling agent, wherein the water-soluble gelling agent is other than a cross-linked polymer of acrylic acid and said water-soluble gelling agent being galactomannan or glucomannan or a combination thereof, (iii) an anti-aggregation agent; (iv) a pH adjusting agent; and (b) water. Prior to use, the composition (a) is admixed with the water (b) to form said debriding formulation characterized by being a homogenous hydrogel having a viscosity in the range of 1,800 Pa·s to 9,350 Pa·s, measured by an absolute viscometer with plate geometry at room temperature, and having a pH ranging from 5.4 to 8.8. The amount of proteins in the debriding formulation ranges from 0.5 % (w/w) to 7 % (w/w) of the total weight of said debriding formulation, wherein said debriding formulation being topically applied to a wound site of a subject in a regimen of up to ten applications during a time period of up to four weeks The debriding formulation is maintained in contact with the wound site for at least four hours per application, thereby achieving debridement of eschar/slough and various forms of devitalized necrotic tissues.

Wound debridement is a key process of wound bed preparation (WBP) and is considered an essential intervention in chronic wound management which may promote wound healing and complete wound closure.

It is known that enzymatic debridement agents available today for the treatment of chronic wounds, such as Santyl^{®} ointment, are applied daily for long periods of time, e.g., for three, six or even twelve months, to achieve eschar removal.

It is now disclosed that applying the debriding formulation of the present invention onto chronic wounds up to 10 times, while maintaining the debriding formulation on the wound site for about 24 hours per application, resulted in essentially complete eschar debridement of the chronic wounds. The debriding formulation for use according to the present invention requires a short term treatment regimen, improve patient compliance, and bring to debridement of chronic or hard to heal wounds within days, i.e., at a faster rate than any enzymatic debridement method known today. Thus, the debriding formulation for use according to the present invention are highly advantageous over known methods of enzymatic wound debridement which are currently being used.

The debriding formulation for use in the debridement of wounds of the present invention comprises, according to some embodiments, the following constituents:
(a) a composition in a dried or lyophilized form comprising:
   (i) a mixture of proteolytic enzymes obtained from bromelain comprising stem bromelain (EC 3.4.22.32) and ananain (EC 3.4.22.31);
   (ii) a water-soluble gelling agent, wherein the water-soluble gelling agent is other than a cross-linked polymer of acrylic acid;
   (iii) an anti-aggregation or anti-agglomeration agent;
   (iv) a pH adjusting agent; and
(b) water,
wherein, prior to use, the composition (a) being admixed with the water (b) to form a debriding formulation characterized by being a homogenous hydrogel having a viscosity in the range of about 2,000 Pa.s (2,000,000 cP) to about 8,500 Pa.s (8,500,000 cP) measured by an absolute viscometer with plate plate geometry at room temperature and having a pH in the range of about 6.0 to about 8.0, wherein the amount of proteins in the debriding formulation ranges from about 0.5% (w/w) to about 7% (w/w) of the total weight of the debriding formulation.

The debriding formulation of the present invention has in some embodiments a pH ranging from about 6.4 to about 8.0, e.g., a pH of about 7.0. At this pH range, the activity of the proteolytic enzymes is essentially maximal. In order to achieve these pHs, the debriding formulation of the present invention comprises a pH adjusting agent, thus enabling obtaining a highly efficacious enzymatic debriding agent.

It is further disclosed that due to the protein nature of the active agents of the debriding formulation, namely a mixture of proteolytic enzymes obtained from bromelain, this mixture tends to form aggregates or agglomerations. In order to prevent aggregate formation, the debriding formulation further comprises an anti-aggregation agent, thus enabling the formation of a homogenous hydrogel.

It is further disclosed that by virtue of its constituents, the debriding formulation of the present invention is adequately viscous to enable, on one hand, the penetration of the proteolytic enzymes to the eschar tissue of chronic wounds so as to effectively debride the non-viable tissue, and, on the other hand, to be localized to the wound site, with essentially no leakage of the debriding formulation from the wound site.

It is also disclosed that due to its constituents, the preparation process of the debriding formulation is simple, ease, and quick, taking only few minutes, e.g., less than two minutes, to mix the constituents and to obtain the homogenous hydrogel. Due to the ease of mixing, the preparation of the debriding formulation of the present invention can be performed by the patient, with no need of assistance by medical personnel. The debriding formulation for use according to the present invention are thus particularly advantageous for elderly patients who have chronic or hard to heal wounds.

According to some embodiments, the wound to be debrided is a chronic wound. According to further embodiments, the chronic wound is selected from the group consisting of a diabetic ulcer, a venous stasis ulcer, an arterial insufficiency ulcer, a chronic pressure ulcer, a chronic post-operative wound, and chronic a post-trauma wound. Each possibility represents a separate embodiment of the invention. According to further embodiments, the chronic wound is a diabetic lower extremity ulcer or a venous leg ulcer.

According to additional embodiments, applying the debriding formulation is performed in a regimen of up to 10 applications, wherein the debriding formulation is maintained in contact with the wound site for about 4-24 hours, such as for about 6 hours, for about 8 hours, for about 10 hours, for about 12 hours, for about 16 hours, for about 24 hours, or for any integer in between per application per day. Each possibility represents a separate embodiment of the invention. According to a certain embodiment, the debriding formulation is maintained in contact with the wound site for about 24 hours per application for up to 10 applications, alternatively for up to 8 applications. According to an exemplary embodiment, the debriding formulation is applied daily for about 24 hours per application for 10 consecutive days.

According to yet further embodiments, applying the debriding formulation is performed in a regimen of up to 10 applications of every other day, wherein the debriding formulation is maintained in contact with the wound site for about 48 hours per application. According to still further embodiments, applying the debriding formulation is performed in a regimen of up to 8 applications of every other day, wherein the debriding formulation is maintained in contact with the wound site for about 48 hours per application.

According to yet further embodiments, applying the debriding formulation is performed in a regimen of three applications per week for up to 10 applications, or for up to 8 applications, wherein the debriding formulation is maintained in contact with the wound site for a time period selected from the group consisting of about 48 hours per application and about 72 hours per application.

According to still further embodiments, the regimen of applying the debriding formulation as defined in any of the above is repeated once, twice or until the wound is completely debrided. Additionally or alternatively, if eschar reoccurs and wound closure is not yet obtained, the regimen is repeated one, two, or more times until eschar is completely debrided.

According to yet further embodiments, the regimen of applying the debriding formulation is followed by a halt of application of at least one day, such as of two days, three days, four days, five days, six days, one week, two weeks, three weeks, four weeks, or more, or any integer in between. Each possibility represents a separate embodiment of the invention.

According to yet further embodiments, the method further comprises a step of washing the wound site after the at least 4 hours of contact with the debriding formulation, such as, for example, after the about 6 hours of contact, after the about 8 hours of contact, after the about 10 hours of contact, after the about 12 hours of contact, after the about 24 hours of contact, after the about 48 hours of contact, or after the about 72 hours of contact of the debriding formulation with the wound site.

According to still further embodiments, the method can further comprise a step of administering to the subject an active agent selected from the group consisting of anesthetic agents, antibacterial agents, antifungal agents, and anti-inflammatory agents. The active agent, such as, for example, the anesthetic agent can be topically applied to the wound site or can be administered orally or parenterally before application of the debriding formulation, concomitant with the application of the debriding formulation, or after application of the debriding formulation.

According to additional embodiments, the amount of proteins in the debriding formulation ranges from about 2 % (w/w) to about 7 % (w/w) of the total weight of the debriding formulation, alternatively from about 1 % (w/w) to about 5 % (w/w) of the total weight of the debriding formulation, further alternatively of about 1 % (w/w), 2 %, 2.5 %, 3 %, 4 %, 5 %, 6 %, or of about 7 % of the total weight of the debriding formulation. Each possibility represents a separate embodiment of the invention. According to a certain embodiment, the amount of proteins in the debriding formulation is of about 2% (w/w) of the total weight of the debriding composition.

The water-soluble gelling agent is, galactomannan or glucomannan. Each possibility represents a separate embodiment of the invention. According to a certain embodiment, the galactomannan is guar gum present in an amount ranging from about 0.25 % (w/w) to about 5 % (w/w) of the total weight of the debriding formulation.

According to additional embodiments, the anti-aggregation agent of the debriding formulation is an oligosaccharide selected from the group consisting of lactose, sucrose, mannitol, and glucose. Each possibility represents a separate embodiment of the invention. According to a certain embodiment, the anti-aggregation agent is lactose present in an amount ranging from about 10 % (w/w) to about 25 % (w/w) of the total weight of the debriring formulation.

According to further embodiments, the pH adjusting agent of the debriding formulation is selected from the group consisting of potassium phosphate, potassium carbonate, sodium phosphate, and sodium carbonate. Each possibility is a separate embodiment of the invention. According to a certain embodiment, the pH adjusting agent is a combination of potassium phosphate dibasic and potassium phosphate monobasic present in an amount ranging from about 2% (w/w) to about 10% (w/w) of the total weight of the debriding formulation.

According to further embodiments, the viscosity of the debriding formulation of the present invention ranges from about 2,000 Pa.s (2,000,000 cP) to about 7,000 Pa.s (7,000,000 cP) alternatively from about 2,400 Pa.s (2,400,000 cP) to about 6,200 Pa.s ( 6,200,000 cP). Each possibility is a separate embodiment of the invention.

According to another embodiment, the pH of the debriding formulation to be used in the method of the present invention ranges from about 6.0 to about 7.0. According to a certain embodiment, the pH is of about 7.0.

According to some embodiments, water is present in an amount ranging from about 55 % (w/w) to about 90 % (w/w) of the total weight of the debriding composition.

According to further embodiments, the debriding formulation of the present invention further comprises an agent selected from the group consisting of anti-foaming agents such as, for example, polyethyleneglycols (PEGs), anti-oxidants, and preservatives. According to one exemplary embodiment, the debriding formulation further comprises PEG.

According to yet further embodiments, the debriding formulation further comprises an active agent selected from the group consisting of anesthetic agents, analgesic agents, anti-inflammatory agents, antibiotic agents, anti-fungal agents, growth factors, and agents promoting healing.

According to some embodiments, the wound to be treated is a chronic wound and the debriding formulation comprises:
(i) the proteolytic enzyme mixture obtained from bromelain comprising stem bromelain (EC 3.4.22.32) and ananain (EC 3.4.22.31), designated throughout the specification and claims active principal ingredient (API);
(ii) guar gum in an amount ranging from about 0.25% (w/w) to about 5% (w/w) of the total weight of the debriding formulation;
(iii) lactose in an amount ranging from about 10% (w/w) to about 25% (w/w) of the total weight of the debriding formulation;
(iv) potassium phosphate in an amount ranging from about 2% (w/w) to about 10% (w/w) of the total weight of the debriding composition; and
(v) water in an amount to complete to 100% (w/w) of the total weight of the debriding formulation,
wherein the amount of proteins in the debriding formulation ranges from about 0.5 % (w/w) to about 7 % (w/w), preferably ranging from about 1 % (w/w) to about 5 % (w/w) of the total weight of the debriding formulation.

According to further embodiments, the wound to be treated is a chronic wound and the debriding formulation comprises:
(i) the proteolytic enzyme mixture obtained from bromelain comprising stem bromelain (EC 3.4.22.32) and ananain (EC 3.4.22.31), designated throughout the specification and claims active principal ingredient (API);
(ii) guar gum in an amount ranging from about 0.25% (w/w) to about 5% (w/w) of the total weight of the debriding formulation;
(iii) lactose in an amount ranging from about 10% (w/w) to about 25% (w/w) of the total weight of the debriding formulation;
(iv) potassium phosphate in an amount ranging from about 2% (w/w) to about 10% (w/w) of the total weight of the debriding composition;
(v) PEG in an amount ranging from about 0.5% (w/w) to about 10% (w/w) of the total weight of the debriding composition; and
(vi) water in an amount to complete to 100% (w/w) of the total weight of the debriding formulation,
wherein the amount of proteins in the debriding formulation ranges from about 0.5 % (w/w) to about 7 % (w/w), preferably ranging from about 1 % (w/w) to about 5 % (w/w) of the total weight of the debriding formulation.

According to a certain embodiment, the debriding formulation of the present invention comprises:

| Ingredient | (%) w/w of formulation |
|---|---|
| API | 2 |
| Guar gum | 3.5 |
| Lactose | 18.05 |
| Potassium phosphate dibasic | 2.5 |
| Potassium phosphate monobasic | 0.8 |
| PEG-3350 | 2 |
| Water for injection | 71.15 |

According to additional embodiments, the debriding formulation to be used in the method of wound debridement of the present invention is prepared by the following steps:
(a) obtaining a composition in a dried or powdered form, the composition comprising:
   (i) the proteolytic enzyme mixture obtained from bromelain;
   (ii) the water-soluble gelling agent;
   (iii) an anti-aggregation agent;
   (iv) a pH adjusting agent; and
(b) admixing, prior to use, the composition (a) with water to form said debriding formulation.

These and other embodiments of the present invention will be better understood in relation to the figures, description, examples and claims that follow.

### BRIEF DESCRIPTION OF THE FIGURES

FIGs. **1A-F** are photographs of chronic wounds induced in pigs. FIG. **1A** shows the chronic wound before treatment and FIGs. **1B** and **1C** show the chronic wound at the 7^{th} and 10^{th} day of treatment, respectively, with the debriding formulation of the present invention. As a control, a chronic wound before treatment (FIG. **1D****)** or after treatment with vehicle only at the 7^{th} and 10^{th} day (FIG. **1E** and **1F****,** respectively) are shown.
FIG. **2** shows the percent clean area after the 10^{th} treatment of chronic wounds induced in pigs as a function of the concentration of the active pharmaceutical ingredient (API) applied to the wound for 24 hours. Wide dash lines indicate confidence intervals (95%) of the model.
FIG. **3** shows the clean area under the curves (AUC) as a function of the concentration of API applied to chronic wounds induced in pigs for 24 hours. Wide dash lines indicate confidence intervals (95%) of the model.
FIG. **4** shows the eschar area under the curves (AUC) as a function of the concentration of API applied to chronic wounds induced in pigs for 24 hours. Wide dash lines indicate confidence intervals (95%) of the model.

### DETAILED DESCRIPTION OF THE INVENTION

The present invention provides means for debridement of a wound and/or for treating a wound and/or for promoting closure of a wound and/or for healing a wound, which may be topically applyied to a wound site of a subject in need of such treatment a therapeutically effective amount of a debriding formulation in a regimen of up to ten applications during a time period of up to four weeks, wherein the debriding formulation is present in the form of a hydrogel comprising: (i) a proteolytic enzyme mixture obtained from bromelain comprising stem bromelain (EC 3.4.22.32) and ananain (EC 3.4.22.31); and (ii) a water-soluble gelling agent, wherein the water-soluble gelling agent is other than a cross-linked polymer of acrylic acid, and wherein said debriding formulation is maintained in contact with the wound site for at least four hours per application. The present invention further provides a debriding formulation in the form of a hydrogel which comprises a proteolytic enzyme mixture obtained from bromelain and a water-soluble gelling agent other than a cross-linked polymer of acrylic acid.

It is now disclosed that applying the debriding formulation of the present invention on a chronic wound induced in pigs for up to 10 applications, when the debriding formulation is maintained in contact with the wound site for 24 hours per application, resulted in essentially complete eschar debridement of the chronic wounds. Similar debridement can be achieved if the debriding formulation is applied to a chronic wound three times a week for up to 10 applications when the debriding composition is maintained in contact with the wound site twice for 48 hours per application and once for 72 hours per application.

### Debriding formulation

The present disclosure provides a debriding formulation comprising a proteolytic enzyme mixture obtained from bromelain as an active ingredient and various excipients.

The term "proteolytic enzyme mixture obtained from bromelain" as used throughout the specification and claims refers to an enzymatic preparation partially purified from bromelain.

The term "bromelain" refers to a protein extract derived from the stems of pineapple plants which can be purchased commercially.

The proteolytic enzyme mixture obtained from bromelain (also termed Debrase^{®} or NexoBrid^{®}) and the preparation thereof are disclosed in WO 2006/054309 and WO 2013/011514. The proteolytic enzyme mixture obtained from bromelain comprises at least two of the cysteine proteases present in bromelain: stem bromelain (EC 3.4.22.32) and ananain (EC 3.4.22.31). The proteolytic mixture can further comprise one or more of the cysteine protease precursors of bromelain such as, for example, ananain (EC 3.4.22.31) precursor, fruit bromelain (EC 3.4.22.33) precursor, and stem bromelain (EC 3.4.22.31) precursor. The proteolytic mixture can further comprise cysteine protease fragments (see, for example, WO 2006/054309), a jacalin-like lectin, and/or bromelain inhibitors. According to a certain embodiment, the proteolytic mixture obtained from bromelain comprises stem bromelain (EC 3.4.22.32), ananain (EC 3.4.22.31), a cysteine protease precursor of bromelain, and a jacalin-like lectin.

The proteolytic enzyme mixture can be obtained by the procedure disclosed in WO 2013/011514. As the last step of the preparation, the proteolytic mixture is lyophilized and stored as a lyophilized powder until use.

The proteolytic enzyme mixture is highly stable and can be stored at 2-8° C for long periods of time, e.g., up to three years. After this period of time, the proteolytic enzyme mixture maintains at least 90% of the original debriding activity which is determined immediately after the preparation process.

The proteolytic enzyme mixture is denoted throughout the specification and claims as the active principal ingredient (API). According to the invention, the amount of proteins, or alternatively the amount of API, in the debriding formulation ranges from about 0.5 % (w/w) to about 7 % (w/w) of the total weight of the debriding formulation. According to additional embodiments, the amount of proteins or API ranges from about 1 % (w/w) to about 5 % (w/w) , such as of about 1 % (w/w), 2 %, 3%, 4%, 5%, 6%, 7% of the total weight of the debriding formulation, or alternatively of about 2 % (w/w) of the total weight of the debriding formulation.

The terms "dry", "dried", "lyophilized" or "powdered" composition as used interchangeably throughout the specification and claims refer to the composition which contains water in an amount of no more than about 5% (w/w) of the total weight of the composition, alternatively water is present in an amount of no more than about 3%, 2%, 1%, 0.5%, or further alternatively no more than about 0.1% (w/w) of the total weight of the composition. According to a certain embodiment, the composition is devoid of water.

The term "hydrogel" as used herein refers to an aqueous composition capable of maintaining a gel-like form.

The term "homogenous" hydrogel means a hydrogel having uniform viscosity (e.g., well mixed throughout).

The excipients of the debriding formulation are all pharmaceutically acceptable. The term "pharmaceutically acceptable" means approved by a regulatory agency of the Federal or a state government or listed in the U. S. Pharmacopeia or other generally recognized pharmacopeia for use in humans.

The term "about" refers to a value which is 10% above or below the indicated value.

According to some embodiments, the excipients of the debriding formulation are water-soluble. The term "water soluble" refers to an agent which typically has solubility in water in the range of 1 gr/ml to 1 gr/30 ml at room temperature.

The water-soluble gelling agent can be a naturally occurring gelling agent, a semi-synthetic gelling agent, and a synthetic gelling agent. The gelling agents according to the present invention do not include cross-linked polymers of acrylic acid.

The water-soluble naturally occurring gelling agent includewater-soluble naturally occurring polysaccharides such as, for example, galactomannans, glucomannansor a combination thereof. Each possibility represents a separate embodiment. Non-limiting examples of galactomannans and glucomannans are guar gum, locust bean gum, xanthan gum, gum acacia, gum tragacanth, gellan gums, and mixtures thereof. Each possibility represents a separate embodiment. According to a certain embodiment, the water-soluble naturally occurring gelling agent is guar gum.

Other biopolymers include, for example chitin, chitosan, collagens, gelatin, glycosaminoglycans such as, for example, heparin, chondroitin sulfate, dermatan sulfate, and heparan sulfate, proteoglycans, fibronectins, and laminins.

Semi-synthetic gelling agents include, but are not limited to, cellulose ethers (e.g. hydroxyethyl cellulose, methyl cellulose, carboxymethyl cellulose, hydroxy propylmethyl cellulose), polyvinylpyrrolidone, polyvinylalcohol, hydroxypropyl guar gum, and the like.

The synthetic gelling agents include, but are not limited to, carboxyvinyl polymers, polyvinylpyrrolidone, polyvinyl acetate polymers, polyvinyl chloride polymers, polyvinylidene chloride polymers and the like.

The debriding composition can further comprise at least one excipient selected from the group consisting of an anti-aggregation agent and a pH adjusting agent.

The anti-aggregation agent suitable for practicing the present invention is any known anti-aggregation agent, such as a water-soluble oligosaccharide, for example, lactose, sucrose, mannitol, sorbitol, and glucose. Each possibility represents a separate embodiment. According to a certain embodiment, the anti-aggregation agent is lactose.

The pH adjusting agent preferably has a pKa of above 6.0. In some embodiments, the pH adjusting agent can be any known pH adjusting agent such as, for example, potassium phosphate, potassium carbonate, sodium carbonate, and sodium phosphate. According to some embodiments, the pH adjusting agent is a combination of potassium phosphate monobasic and potassium phosphate dibasic present in an amount ranging from about 2% (w/w) to about 10% (w/w) of the total weight of the debriding formulation. It is now disclosed that higher amounts of potassium phosphate monobasic and potassium phosphate dibasic in the debriding formulation cause bleeding at the application site. It is therefore disclosed that if the pH adjusting agent is a combination of potassium phosphate monobasic and potassium phosphate dibasic, their total amount are preferably not higher than about 10% of the total weight of the debriding formulation in order to achieve efficient debridement without undesirable bleeding.

The composition can further comprise an anti-foaming agent. Anti-foaming agents are known in the art and include, but not limited to, polyethylene glycols, e.g., PEG-1450, PEG-3350, and the like. The composition can further comprise a preservative such as, for example, benzyl alcohol, parabens, methyl- or propylhydroxybenzoates; and/or an antioxidant such as, for example, ascorbic acid, dihydroquinone, butylated hydroxytoluene and dithiothreitol.

The composition can further comprise an anesthetic agent, an antibacterial agent, an antifungal agent, an anti-inflammatory agent, an analgesic agent, a growth factor and/or an agent promoting healing.

The anesthetic agents include, but are not limited to, amethocaine (tetracaine), lignocaine (lidocaine), xylocaine, bupivacaine, prilocaine, ropivacaine, benzocaine, mepivocaine, cocaine. Each possibility represents a separate embodiment.

The antibacterial agents include, but are not limited to, amanfadine hydrochloride, amanfadine sulfate, amikacin, amikacin sulfate, amoglycosides, amoxicillin, ampicillin, amsamycins, bacitracin, beta-lactams, candicidin, capreomycin, carbenicillin, cephalexin, cephaloridine, cephalothin, cefazolin, cephapirin, cephradine, cephaloglycin, chilomphenicols, chlorhexidine, chloshexidine gluconate, chlorhexidine hydrochloride, chloroxine, chlorquiraldol, chlortetracycline, chlortetracycline hydrochloride, ciprofloxacin, circulin, clindamycin, clindamycin hydrochloride, clotrimazole, cloxacillin, demeclocycline, diclosxacillin, diiodohydroxyquin, doxycycline, ethambutol, ethambutol hydrochloride, erythromycin, erythromycin estolate, erhmycin stearate, farnesol, floxacillin, gentamicin, gentamicin sulfate, gramicidin, giseofulvin, haloprogin, haloquinol, hexachlorophene, iminocylcline, iodochlorhydroxyquin, kanamycin, kanamycin sulfate, lincomycin, lineomycin, lineomycin hydrochloride, macrolides, meclocycline, methacycline, methacycline hydrochloride, methenine, methenamine hippurate, methenamine mandelate, methicillin, metonidazole, miconazole, miconazole hydrochloride, minocycline, minocycline hydrochloride, mupirocin, nafcillin, neomycin, neomycin sulfate, netimicin, netilmicin sulfate, nitrofurazone, norfloxacin, nystatin, octopirox, oleandomycin, orcephalosporins, oxacillin, oxyteacline, oxytetracycline hydrochloride, parachlorometa xylenol, paromomycin, paromomycin sulfate, penicillins, penicillin G, penicillin V, pentamidine, pentamidine hydrochloride, phenethicillin, polymyxins, quinolones, streptomycin sulfate, tetracycline, tobramycin, tolnaftate, triclosan, trifampin, rifamycin, rolitetracycline, silver salts, spectinomycin, spiramycin, struptomycin, sulfonamide, tetracyclines, tetracycline, tobramycin, tobramycin sulfate, triclocarbon, triclosan, trimethoprim-sulfamethoxazole, tylosin, vancomycin, and yrothricin. Each possibility represents a separate embodiment.

The antifungal agents include, but are not limited to, nystatin, clotrimazole, miconazole, ketoconazole, fluconazole, thiabendazole, econazole, clomidazole, isoconazole, tiabendazole, tioconazole, sulconazole, bifonazole, oxiconazole, fenticonazole, omoconazole, sertaconazole, and flutrimazole. Each possibility represents a separate embodiment.

The anti-inflammatory agent can be non-steroidal, steroidal, or a combination thereof. Non limiting examples of non-steroidal anti-inflammatory agents include oxicams, such as piroxicam, isoxicam, tenoxicam, sudoxicam; salicylates, such as aspirin, disalcid, benorylate, trilisate, safapryn, solprin, diflunisal, and fendosal; acetic acid derivatives, such as diclofenac, fenclofenac, indomethacin, sulindac, tolmetin, isoxepac, furofenac, tiopinac, zidometacin, acematacin, fentiazac, zomepirac, clindanac, oxepinac, felbinac, and ketorolac; fenamates, such as mefenamic, meclofenamic, flufenamic, niflumic, and tolfenamic acids; propionic acid derivatives, such as ibuprofen, naproxen, benoxaprofen, flurbiprofen, ketoprofen, fenoprofen, fenbufen, indopropfen, pirprofen, carprofen, oxaprozin, pranoprofen, miroprofen, tioxaprofen, suprofen, alminoprofen, and tiaprofenic; pyrazoles, such as phenylbutazone, oxyphenbutazone, feprazone, azapropazone, and trimethazone. Extracts of these non-steroidal anti-inflammatory agents may also be employed. Each possibility represents a separate embodiment.

Non-limiting examples of steroidal anti-inflammatory drugs include corticosteroids such as hydrocortisone, hydroxyl-triamcinolone, alpha-methyl dexamethasone, dexamethasone-phosphate, beclomethasone dipropionates, clobetasol valerate, desonide, desoxymethasone, desoxycorticosterone acetate, dexamethasone, dichlorisone, diflorasone diacetate, diflucortolone valerate, fluadrenolone, fluclorolone acetonide, fludrocortisone, flumethasone pivalate, fluosinolone acetonide, fluocinonide, flucortine butylesters, fluocortolone, fluprednidene (fluprednylidene) acetate, flurandrenolone, halcinonide, hydrocortisone acetate, hydrocortisone butyrate, methylprednisolone, triamcinolone acetonide, cortisone, cortodoxone, flucetonide, fludrocorisone, difluorosone diacetate, fluradrenolone, fludrocortisone, diflurosone diacetate, fluradrenolone acetonide, medrysone, amcinafel, amcinafide, betamethasone and the balance of its esters, chloroprednisone, chlorprednisone acetate, clocortelone, clescinolone, dichlorisone, diflurprednate, flucloronide, flunisolide, fluoromethalone, fluperolone, fluprednisolone, hydrocortisone valerate, hydrocortisone cyclopentylpropionate, hydrocortamate, meprednisone, paramethasone, prednisolone, prednisone, beclomethasone dipropionate, and triamcinolone. Each possibility represents a separate embodiment.

Analgesic agents include, but are not limited to, codeine, hydrocodone, oxycodone, fentanyl, and propoxyphene. Each possibility represents a separate embodiment.

The growth factors include, but are not limited to, epidermal growth factors, fibroblast growth factors, insulin-like growth factors, and the like.

Agents promoting healing include, but are not limited to, hyaluronic acid and the like.

The viscosity of the gel formulations of the present invention is measured by an absolute viscometer with plate plate geometry to calculate the viscosity of the gel formulations described herein. The viscosity ranges referred to herein are all measured at room temperature.

According to the principles of the present invention, the composition (a) which is present in a dry or powdered form and the water (b) can be placed in a first compartment and a second compartment, respectively, of a single container or can be placed in two separate containers. Before use, the composition (a) and the water (b) are admixed to form the debriding formulation.

The debriding formulations of the present invention are of low bacterial bioburden, and therefore the formulations of the present invention reduce the risk of further contaminating the wound site. According to some embodiments, the debriding formulations are sterile.

The debriding formulation of the invention comprises:
(a) a composition in a dried or powdered form comprising:
   (i) a proteolytic enzyme mixture obtained from bromelain comprising stem bromelain (EC 3.4.22.32) and ananain (EC 3.4.22.31);
   (ii) a water-soluble gelling agent, wherein the water-soluble gelling agent is other than a cross-linked polymer of acrylic acid, said water-soluble gelling agent being galactomannan or glucomannan or a combination thereof;
   (iii) an anti-aggregation agent;
   (iv) a pH adjusting agent; and
(b) water,
wherein the composition (a) being admixed with the water (b) to form a debriding formulation characterized by being a homogenous hydrogel having a viscosity in the range of about 2,000 Pa.s (2,000,000 cP) to about 8,500 Pa.s (8,500,000 cP) and a pH ranging from about 6.0 to about 8.0, and wherein the amount of proteins in the debriding formulation ranges from about 0.5 % (w/w) to about 7 % (w/w) of the total weight of the debriding formulation.

According to some embodiments, the debriding formulation comprises:
(a) a composition in a dried or powdered form, present in a first compartment of a container or in a first container, the composition comprising:
   (i) the proteolytic enzyme mixture obtained from bromelain comprising stem bromelain (EC 3.4.22.32) and ananain (EC 3.4.22.31);
   (ii) guar gum in an amount ranging from about 0.25% (w/w) to about 5% (w/w) of the total weight of the debriding formulation;
   (iii) lactose in an amount ranging from about 10% (w/w) to about 25% (w/w) of the total weight of the debriding formulation;
   (iv) a pH adjusting agent; and
(b) water in an amount ranging from about 55% (w/w) to about 90% (w/w), present in the second compartment of the container or in the second container.

It is to be understood that the debriding formulations of the present invention are formulated as gels, i.e., hydrogels, and as such are applied on to the wound site. Preferably, the debriding formulations are not patch formulations. According to some embodiments, the formulations are devoid of adhesive agents, and thus the formulations are non adhesive.

### Uses of the debriding formulation

The formulation for use in debridement of a skin wound and/or for use in treating a skin wound may be topically applyied to a wound site of a subject in need of such treatment in a therapeutically effective amount of a debriding formulation in a regimen of up to ten applications during a time period of up to four weeks, wherein the debriding formulation present in the form of a hydrogel comprising: (i) a proteolytic enzyme mixture obtained from bromelain comprising stem bromelain (EC 3.4.22.32) and ananain (EC 3.4.22.31); and (ii) a water-soluble gelling agent, wherein the water-soluble gelling agent is other than a cross-linked polymer of acrylic acid, and wherein said debriding formulation is maintained in contact with the wound site for at least four hours per application.

According to some embodiments, the wound is a chronic or hard to heal wound.

The terms "chronic wound", "chronic skin wound" or a "hard to heal wound" as used interchangeably throughout the specification and claims refer to a wound that has failed to proceed through an orderly and timely series of events to produce a durable structural, functional, and/or cosmetic closure as wounds do. Wounds that do not heal within one month are considered chronic wounds.

According to some embodiments, the chronic wound is selected from the group consisting of a diabetic ulcer, a venous stasis ulcer, an arterial insufficiency ulcer, a chronic pressure ulcer, a chronic post-operative and a chronic post trauma wound. Each possibility represents a separate embodiment. According to further embodiments, the chronic wound is a diabetic lower extremity ulcer or a venous leg ulcer.

The term "debridement of a wound" as used herein refers to the removal of nonviable tissue: necrotic eschar, slough or fibrin, foreign material, and bacteria/biofilm from a wound. Necrotic eschar is a thin or thick, leathery, devitalized, black, brown or tan tissue, whereas slough and biofilm are exudative, white or yellow-greenish mottled, tenuous tissue on the wound bed. Necrotic tissue, foreign material and bacteria impede the body's attempt to heal by producing or stimulating the production of metalloproteases that interfere with the local wound-healing process. This hostile environment allows bacteria to proliferate, further colonize the wound within the exudates, debris, and purulent discharges ("slough") that cover the wound bed. In addition, the bacteria secrete structural products that together with the slough form the biofilm, thus protect their colonies from potential destruction. The bacteria produce their own wound-inhibiting enzymes and, more significantly, consume much of the scarce, available local resources that are necessary for wound healing.

According to some embodiments, debridement of a wound refers to removal of at least 50%, alternatively of at least 75% of the non-viable tissue which is present prior to treatment. Each possibility represents a separate embodiment of the invention. According to certain embodiments, debridement of a wound refers to removal of at least 90%, or of at least 95%, and preferably of 100% of the non-viable tissue which is present prior to treatment; such debridement, namely of 90% or more of the non-viable tissue present prior to treatment is referred throughout the specification and claims as "complete debridement of a wound".

In chronic or hard to heal wounds several different factors may play an important role. Exposed surfaces such as bone, tendons, fascia or even fat do not support cellular proliferation and they dry and become foreign bodies such as synthetic implants. Any interference with local blood supply (arterial, venous, lymphatic, pressure etc.) may cause a wound to become hard to heal and chronic. Granulation tissue may become recalcitrant, atrophic, lose its rich vascular matrix, become darker and opaque in color and will not take any part in the wound healing and closure processes.

The term "wound bed preparation" as used herein refers to a wound bed which results from a proper debridement in order to accelerate endogenous healing or to facilitate the effectiveness of other therapeutic measures. It is a process of debriding, removing various "burdens" within both the wound and the patient that impede healing. Burdens within the wound include exudate, bacteria, biofilm and necrotic/cellular debris. The overall health status of the patient is important to the healing process. In chronic or hard to heal wounds complete removal of the offending eschar, slough or biofilm may result in a clean wound bed, yet such a wound bed may still be inadequate for future healing if the patient's systemic or the extremity's condition cannot support it.

A wound bed prepared for healing is one without eschar, slough, fibrin or biofilm that also has a viable bed of healthy tissues and/or healthy granulation tissue (level >7 in the granulometer scale) that will allow the wound to close spontaneously by scarring and contracture-epithelialization (optionally using modalities such as biological dressings, wound-healing enhancing dressings, synthetic wound dressings, vacuum or ozone wound healing systems) over the viable, clean bed or will support autologous STSG (Split Thickness Skin Graft) or skin allografting.

The term "wound closure" refers to the process of regenerating the covering cell layers of a tissue. Thus, promoting wound closure means creating a positive effect in the regeneration of the covering cell layers. The positive effect can be an acceleration of the regeneration process or a decrease of the damaged area of the wound. Wound closure is also defined as full epithelialization without drainage, and without need for additional dressing, confirmed at two consecutive study visits 2 weeks apart

The term "therapeutically effective amount" is that amount of the proteolytic enzyme mixture which is sufficient to provide a beneficial effect to the subject to which the composition is administered.

According to some embodiments, the debriding formulation can be applied to a wound site up to 10 applications, wherein the debriding formulation is maintained in contact with the wound site for at least four hours per application per day.

According to additional embodiments, the debriding formulation can be applied in a regimen of up to 10 times to a wound site, wherein the debriding formulation is maintained in contact with the wound site for about 24 hours per application. Thus, the debriding formulation can be applied daily for up to 10 consecutive days so as to be maintained in contact with the wound site for about 24 hours per application or can be applied continuously 1, 2, 3, 4, 5, 6, 7, 8, or 9 applications for about 24 hours per application with a halt of application in between of one day or more as required.

According to additional embodiments, the debriding formulation can be applied in a regimen of up to 10 times to a wound site, wherein the debriding formulation is maintained in contact with the wound site for about 48 hours per application. Thus, the debriding formulation can be applied every other day for up to 20 consecutive days or can be applied 1, 2, 3, 4, 5, 6, 7, 8, or 9 times every other day with a halt of application in between of one day or more as required.

According to further embodiments, the debriding formulation can be applied in a regimen of up to 10 times to a wound site, three times a week, wherein the debriding formulation is maintained in contact with the wound site for a duration selected from the group consisting of 48 hours per application and 72 hours per application.

According to additional embodiments, the debriding formulation can be applied in a regimen of up to 10 times to a wound site, wherein the debriding formulation is maintained in contact with the wound site for about 72 hours per application.

According to certain embodiments, the debriding formulation is maintained in contact with the wound site up to about 72 hours per application.

After the contact of the debriding formulation with the wound site for the indicated application time, such as after at least 4 hours treatment, or after the 24 hours treatment, or after the 48 hours treatment, or after the 72 hours treatment, the wound site can be washed. Thus, the methods using the debriding formulation of the present invention can further comprise a step of washing the wound site after said contact, prior to a subsequent application of the debriding formulation. If a halt of application is performed, the wound site can be covered with a moist dressing such as moist saline gauze.

According to some embodiments, the methods using the debriding formulation of the present invention can further comprise a step of covering the debriding formulation with an occlusive layer or dressing to maintain or hold the composition at the wound site.

According to additional embodiments, the method using the debriding formulation of the present invention can further comprise a step of protecting the wound edges and the peri-wound skin during debridement.

The ranges of numerical values indicated throughout the specification and claims include any integer in between.

It is to be understood that the regimens defined in any of the above can be repeated one, two, three or more times until the eschar/necrotic tissue is completely debrided, optionally with a halt of application. The halt of application can be of days, weeks or months. The regimen of application of the debriding formulation can be repeated as necessary to debride eschar. If eschar reoccurs, the regimen of application of the debriding formulation can be repeated as necessary to debride eschar.

The present disclosure encompasses combination therapy wherein the methods using the debriding formulation of the present invention can be combined with known debridement methods, such as, surgical or sharp debridement. According to some embodiments, the methods using the debriding formulation of the present invention can be performed prior to surgical or sharp debridement. Alternatively, the methods using the debriding formulation of the present invention can be performed after surgical or sharp debridement.

According to some embodiments, the amount of API applied ranged between about 0.1 gr to about 2 gr of the sterile lyophilized proteolytic enzyme mixture per 100 cm² of wound surface. According to additional embodiments, the amount of hydrogel applied to a wound site is of about 20 gr per 100 cm² of wound surface.

### EXAMPLE 1

### Gel formulation

The following debriding formulations were developed:

| Ingredient | % (w/w) in formulation | Function |
|---|---|---|
| Proteolytic enzyme mixture obtained from bromelain (API) | 5^{∗} | Active ingredients (API) |
| Guar gum | 3.5 | Gelling agent |
| Lactose | 15^{∗∗} | Anti-agglomeration agent |
| Potassium phosphate dibasic | 2.5 | pH adjusting agent |
| Potassium phosphate monobasic | 0.8 | pH adjusting agent |
| PEG-3350 | 2 | Anti-foaming agent |
| Water for injection | Complete to 100% | |

| | | |
|---|---|---|
| ^{∗} Other amounts of API (w/w) which were evaluated: 0.1%; 0.5%; 1%; and 2%. ^{∗∗} The amount of lactose was adjusted according to the amount of API. | | |

The debriding formulations were prepared by admixing the dried or powdered composition which contained API, guar gum, lactose, potassium phosphate dibasic and monobasic, and PEG-3350, with water to form the hydrogel having a homogenous appearance and which has a viscosity ranging from 2,400 Pa.s (2,40,000 cP ) to 6,200 Pa.s (6,200,000 cP).

### EXAMPLE 2

### Debridement of eschar by the gel formulation

The aim of this study was to determine the dose of the active ingredients in the gel formulation which provides maximal efficacy of eschar debridement of chronic wounds.

A chronic wound model was established in crossbred domestic pigs.

Prior to application of the gel formulation, wound edges were protected with thick layer of Vaseline. Each wound site received ~2 g of the gel formulation to cover the wound for 24 hours, and bandaged with non absorbing dressing. Each wound was photographed before and after each application. The following doses were examined: placebo (0%), 0.1%, 0.5%, 1%, 2%, 5%.

This procedure was followed for up to 11 consecutive daily treatments or until clean wound bed was achieved. This period was denoted the "Treatment period". The treatment period was followed by two weeks "recovery period" with no treatments. In the recovery period the wounds were photographed 3 times a week.

The wound area, clean area and eschar volume were evaluated visually, measured by ImageJ software (NIH, MD, USA) and analyzed by JMP statistical software (SAS Inc., NC, USA).

On the first treatment day, all wounds were covered by a full eschar. The eschar composed of 2 distinct areas:
a. the center of the wound, which was covered with thin eschar layer;
b. the wound edges, which were characterized by fully necrotic tissue.

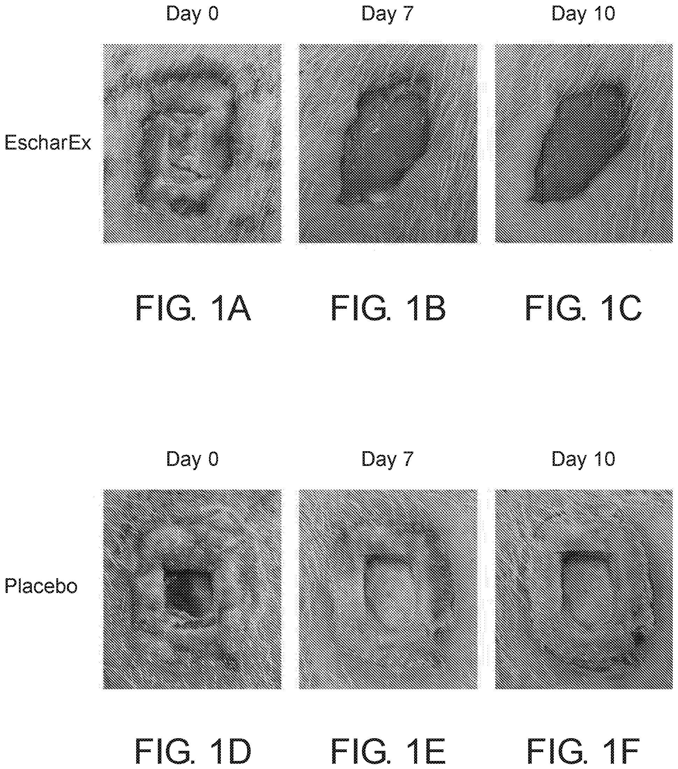

Representative photographs of the wound before treatment and after treatment 7 or 10 days with the gel formulation or with the gel vehicle are shown in FIGs. **1A-1F****.**

At the beginning of the treatment period, the chronic wounds already developed eschar. FIGs. **1A** and **1D** are representative photographs showing the chronic wound before treatment. The eschar was composed of two distinct areas: the center of the wound where the skin was fully excised, and the wound edges. In the center, a thin eschar layer was developed, while in the edges, the eschar was composed of necrotic skin. In chronic wounds treated with the gel formulation containing API, the eschar softened and gradually dissolved from treatment to treatment until the dissolution at the circumference caused the eschar to wholly disconnect from the viable tissue (FIGs. **1B** and **1C****).** In contrast, chronic wounds treated with the gel vehicle barely changed their appearance and consistency during the treatment period (FIG. **1E** and **1F**).

The clean area was calculated as percent from total wound size. The volume of the eschar was calculated as percentage from the eschar volume just before the first treatment, taking into account both the area and the thickness of eschar.

The efficacy of the treatments was evaluated by measuring the area under the curve (AUC) where the x axis is the day of treatment and y axis is the percent clean area or percent eschar volume. The more effective the treatment, the larger is the area under the curve for percent clean area and the smaller the area under the curve for eschar volume.

To assess the irritation caused by the formulation, five blinded assessors scored each wound according to the photographs of the entire experiment.

**Table 1. Summary of the debridement results**

| Parameter (Y) | Gel formulation | Placebo |
|---|---|---|
| % clean area | 82 | 52 |
| % eschar from day 0 | 7 | 31 |
| AUC clean | 454 | 274 |
| AUC eschar | 531 | 850 |

### Percent clean area out of the wound area at the end of the treatment period:

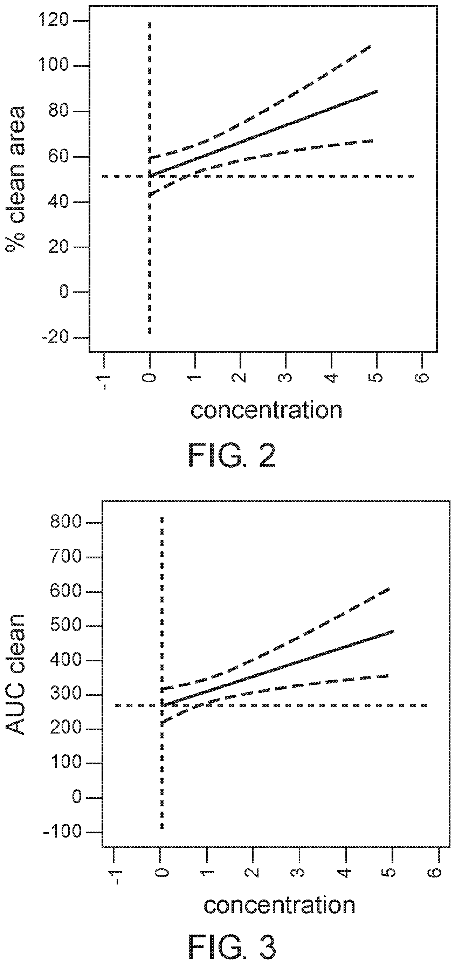

The percent of clean area at the end of treatments out of the initial wound area was found to be significantly dependent on the amount of API. The dependency on the amount of API was linear (FIG. **2**).

At a dose of 5% of API in the gel formulation, an average of 82% of the wound was clean.

### Percent of eschar out of the initial amount of the wound at the end of the treatment period:

The percent of eschar at the end of treatments out of the initial amount was found to be significantly dependent on the amount of API. The dependency on the amount of API was linear.

At a dose of 5% of API in the gel formulation, an average of 93% of the eschar was removed.

### The area under the curve (AUC) where the x axis is day of treatment and the y axis is the percent clean area:

This parameter shows the cleaning efficacy of the treatments: the more effective the treatments are, the larger the AUC. The AUC of percent clean area during treatments was significantly dependent on the amount of API. As shown in FIG. **3****,** the dependency of AUC percent clean area on API amount was linear.

### The area under the curve (AUC) where the x axis is day of treatment and the y axis is the percent of eschar out of the initial amount of eschar:

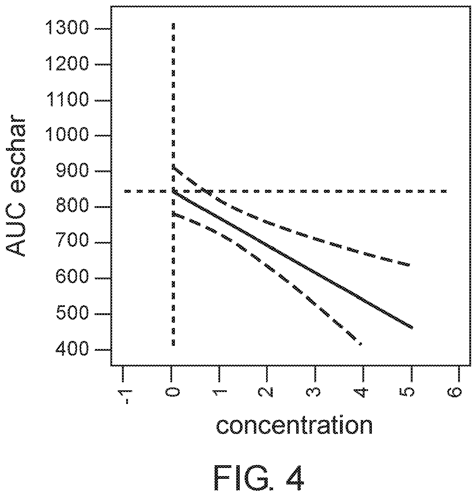

This parameter shows the eschar removal efficacy of the treatments: the more effective the treatments are, the smaller the AUC. This parameter was found to be significantly dependent on the mount of API. The dependency on API amount was linear (FIG. **4**).

Taken together, these results indicated that the effect of API was does and time dependent.

### Irritation

Five treatment-blinded assessors scored each wound based on the photographs of the wounds through the entire experiment. The irritation caused by the placebo was found to be significantly lower than that of the treatment groups. Irritation was dependent on API amount and disappeared completely after a day or two in the follow up period in all treatments.

### EXAMPLE 3

### Efficacy and safety of API in the gel formulation - Clinical study

The aim of this study is to assess the safety and the efficacy of two doses: 2% (w/w) and 5% (w/w) of the gel formulation disclosed herein above in Example 1, also designated EX-02, compared to placebo in debridement of chronic venous leg ulcers and of diabetic lower extremity ulcers.

The study is a multicenter, prospective, randomized, placebo controlled, double-blind, international study.

Adults with >50% necrotic/slough/fibrin non-viable tissue on a chronic wound (venous leg ulcer, diabetic lower extremity ulcer) between 3 cm² and 200 cm² (surface area) are enrolled into the study.

Patients are randomized to EX-02 Low dose (2% w/w), EX-02 high dose (5% w/w), or Placebo treatment group. Treatment is performed three times a week up to 10 applications (up to 10 visits) or until complete debridement is achieved, whichever occurs first. The duration of each application is 24±2 hours or three times a week, namely 48±4 hours and 72±4 hours per application. Following each application the wound is washed, photographed and assessed for wound size and removal of nonviable tissue (by digital planimetry software) and change in granulation tissue, wound status, and safety parameters. The 24 hour treatments are performed successively during week days. During weekends the wound are dressed with moist-to-moist saline gauze.

Following completion of the debridement treatment period, patients are treated according to standard procedures and evaluated (wound assessments) once a week until complete wound closure for up to 12 weeks from last application (up to 12 visits). For patients who achieved wound closure, additional 3 monthly follow- up visits of wound closure confirmation are conducted; the first monthly visit is performed 2 weeks after reaching wound closure. For patients who didn't achieve wound closure during the 12-weeks follow-up visits, only the 3-months follow-up visit (week 30) is conducted. The placebo is prepared as a powder of the excipients only and water for the preparation of a gel.

The following endpoints are evaluated and compared between EX-02 and Placebo for all wounds:

### Primary Endpoint

Incidence of complete debridement (non-viable tissue removal) at the end of the debridement period (up to 8 treatment days)

### Secondary Endpoints

1. Time to achieve complete debridement (within up to 8 treatments);
2. Number of applications/treatment days to achieve complete debridement;
3. Assessment of changes in wound debridement status during treatment period : percentage reduction in non viable tissue (daily, during 8 treatments);
4. Time to achieve complete granulation (up to 12 weeks);
5. Incidence of complete granulation (on week 12);
6. Percent of change in granulation tissue over time (weekly, during baseline-12 weeks);
7. Incidence of complete wound closure (up to 12 weeks). Wound closure is defined as full epithelialization without drainage, and without need for additional dressing, confirmed at two consecutive study visits 2 weeks apart;
8. Time to complete wound closure (up to 12 weeks);
9. Wound area reduction: percentage reduction in wound size over time (weekly, from baseline up to 12 weeks).
10. Incidence of infection.

It will be appreciated by persons skilled in the art that the present invention is not limited by what has been particularly shown and described herein above. Rather the scope of the invention is defined by the claims that follow.

## Claims

1. A debriding formulation for use in debridement of a wound, the debriding formulation comprising:
(a) a composition in a dry or powdered form comprising:
(i) a proteolytic enzyme mixture obtained from bromelain comprising stem bromelain (EC 3.4.22.32), and ananain (EC 3.4.22.31);
(ii) a water-soluble gelling agent, wherein the water-soluble gelling agent is other than a cross-linked polymer of acrylic acid, and wherein said water-soluble gelling agent is galactomannan or glucomannan or a combination thereof;
(iii) an anti-aggregation agent;
(iv) a pH adjusting agent; and
(b) water,
wherein, prior to use, the composition (a) being admixed with the water (b) to form said debriding formulation **characterized by** being a homogenous hydrogel having a viscosity in the range of 1,800 Pa·s (1,800,000 centipoise (cP)) to 9,350 Pa·s (9,350,000 cP), measured by an absolute viscometer with plate plate geometry at room temperature, and having a pH ranging from 5.4 to 8.8,
wherein the amount of proteins in the debriding formulation ranges from 0.5 % (w/w) to 7 % (w/w) of the total weight of said debriding formulation,
wherein said debriding formulation being topically applied to a wound site of a subject in a regimen of up to ten applications during a time period of up to four weeks, and
wherein the debriding formulation being maintained in contact with the wound site for at least four hours per application.

2. The debriding formulation for use according to claim 1,
wherein the wound is a chronic wound, preferably wherein the chronic wound is selected from the group consisting of a diabetic ulcer, a venous stasis ulcer, an arterial insufficiency ulcer, a chronic pressure ulcer, a chronic post-operative wound, and a chronic post-trauma wound.

3. The debriding formulation for use according to any one of claims 1 and 2,
wherein the debriding formulation being applied in a regimen of up to 10 applications, and wherein said debriding formulation being maintained in contact with the wound site for a time period selected from the group consisting of 24 hours per application, 48 hours per application, and 72 hours per application.

4. The debriding formulation for use according to claim 3,
wherein the regimen is repeated once, twice, or as needed until the wound being completely debrided.

5. The debriding formulation for use according to any one of claims 1 to 4,
wherein the amount of proteins in said debriding formulation ranges from 1 % (w/w) to 5 % (w/w) of the total weight of the debriding formulation; preferably the amount of proteins in the debriding formulation is of 2 % (w/w) of the total weight of the debriding formulation; or
wherein the galactomannan is guar gum present in an amount ranging from 0.25 % (w/w) to 5 % (w/w) of the total weight of the debriding formulation; or
wherein the anti-aggregation agent is an oligosaccharide, preferably the oligosaccharide is selected from the group consisting of lactose and sucrose, more preferably the oligosaccharide is lactose present in an amount ranging from 10 % (w/w) to 25 % (w/w) of the total weight of the debriding formulation; or
wherein the anti-aggregation agent is selected from mannitol and glucose; or
wherein the pH adjusting agent is potassium phosphate present in an amount ranging from 2 % (w/w) to 10 % (w/w) of the total weight of the debriding formulation, preferably the potassium phosphate is a combination of potassium phosphate dibasic and potassium phosphate monobasic, more preferably the pH of the debriding formulation ranges from 6.0 to 7.0; or
wherein water is present in an amount ranging from 55 % (w/w) to 90 % (w/w) of the total weight of the debriding formulation.

6. The debriding formulation for use according to any one of claims 1 to 5, wherein said debriding formulation further comprises an agent selected from the group consisting of anti-foaming agents, anti-oxidants, and preservatives.

7. The debriding formulation for use according to any one of claims 1 to 6, wherein said debriding formulation further comprises an active agent selected from the group consisting of anesthetic agents, anti-inflammatory agents, antibiotic agents, anti-fungal agents, analgesic agents, growth factors and agents promoting healing.

8. The debriding formulation for use according to any one of claims 1 to 7, wherein said debriding formulation comprises:
(i) the proteolytic enzyme mixture, wherein the amount of proteins in the debriding formulation ranges from 1 % (w/w) to 5 % (w/w) of the total weight of the debriding formulation;
(ii) guar gum in an amount ranging from 0.25 % (w/w) to 5 % (w/w) of the total weight of the debriding formulation;
(iii) lactose in an amount ranging from 10 % (w/w) to 25 % (w/w) of the total weight of the debriding formulation;
(iv) potassium phosphate in an amount ranging from 2 % (w/w) to 10 % (w/w) of the total weight of the debriding formulation; and
(v) water in an amount to complete to 100% (w/w) of the total weight of the debriding formulation.

9. The debriding formulation for use according to claim 8, wherein said debriding formulation comprises:
| Ingredient | (%) w/w of formulation |
|---|---|
| API | 2 |
| Guar gum | 3.5 |
| Lactose | 18.05 |
| Potassium phosphate dibasic | 2.5 |
| Potassium phosphate monobasic | 0.8 |
| PEG-3350 | 2 |
| Water for injection | 71.15 |

10. The debriding formulation for use according to claim 1, wherein said debriding formulation is prepared by the following steps:
(a) obtaining a composition in a dry or powdered form which comprises:
(i) the proteolytic enzyme mixture obtained from bromelain;
(ii) the water-soluble gelling agent;
(iii) the anti-aggregation agent;
(iv) the pH adjusting agent; and
(b) admixing, prior to use, the composition (a) with water to form the debriding formulation.

11. A debriding formulation comprising:
(a) a composition in a dried or lyophilized form comprising:
(i) a proteolytic enzyme mixture obtained from bromelain comprising stem bromelain (EC 3.4.22.32) and ananain (EC 3.4.22.31);
(ii) a water-soluble gelling agent, wherein the water-soluble gelling agent is other than a cross-linked polymer of acrylic acid, and wherein the water-soluble gelling agent is a galactomannan or glucomannan or a combination thereof;
(iii) an anti-aggregation agent;
(iv) a pH adjusting agent; and
(b) water,
wherein, prior to use, the composition (a) being admixed with the water (b) to form a debriding formulation **characterized by** being a homogenous hydrogel having a viscosity in the range of 1,800 Pa·s (1,800,000 centipoise (cP)) to 9,350 Pa s (9,350,000 cP), measured by an absolute viscometer with plate plate geometry at room temperature, and having a pH ranging from 5.4 to 8.8, and
wherein the amount of proteins in the debriding formulation ranges from 0.5 % (w/w) to 7 % (w/w) of the total weight of the debriding formulation.

12. The debriding formulation according to claim 11 comprising:
(a) a composition in a dried or lyophilized form, present in a first compartment of a container or in a first container, the composition comprising:
(i) a proteolytic enzyme mixture obtained from bromelain comprising stem bromelain (EC 3.4.22.32) and ananain (EC 3.4.22.31), wherein the amount of proteins in the debriding formulation ranges from 1 % (w/w) to 5 % (w/w) of the total weight of the debriding formulation;
(ii) guar gum in an amount ranging from 0.25 % (w/w) to 5 % (w/w) of the total weight of the debriding formulation;
(iii) lactose in an amount ranging from 10% (w/w) to 25 % (w/w) of the total weight of the debriding formulation;
(iv) a pH adjusting agent; and
(b) water in an amount of 55 % (w/w) to 90 % (w/w) present in a second compartment of the container or in a second container,
wherein, prior to use, the composition (a) being admixed with the water (b) to form said debriding formulation.

13. The debriding formulation according to claim 12 comprising:
| Ingredient | (%) w/w of formulation |
|---|---|
| API | 2 |
| Guar gum | 3.5 |
| Lactose | 18.05 |
| Potassium phosphate dibasic | 2.5 |
| Potassium phosphate monobasic | 0.8 |
| PEG-3350 | 2 |
| Water for injection | 71.15 |

## Patentansprüche

1. Debridement-Formulierung zur Verwendung beim Debridement einer Wunde, worin die Debridement-Formulierung umfasst:
(a) eine Zusammensetzung in trockener oder pulverisierter Form, umfassend:
(i) eine aus Bromelain gewonnene proteolytische Enzymmischung, die Stamm-Bromelain (EC 3.4.22.32) und Ananain (EC 3.4.22.31) umfasst;
(ii) ein wasserlösliches Geliermittel, worin das wasserlösliche Geliermittel kein vernetztes Acrylsäurepolymer ist, und worin das wasserlösliche Geliermittel Galaktomannan oder Glukomannan oder eine Kombination davon ist;
(iii) ein Anti-Aggregationsmittel;
(iv) ein Mittel zur Einstellung des pH-Werts; und
(b) Wasser,
worin die Zusammensetzung (a) vor der Verwendung mit dem Wasser (b) gemischt wird, um die Debriding-Formulierung zu bilden, die **dadurch gekennzeichnet ist, dass** sie ein homogenes Hydrogel mit einer Viskosität im Bereich von 1.800 Pa-s (1.800.000 Centipoise (cP)) bis 9.350 Pa-s (9.350.000 cP) ist, gemessen mit einem Absolutviskosimeter mit Plattengeometrie bei Raumtemperatur, und einen pH-Wert im Bereich von 5,4 bis 8,8 aufweist,
worin die Menge an Proteinen in der Debriding-Formulierung von 0,5 Gew.-% bis 7 Gew.-% des Gesamtgewichts der Debriding-Formulierung reicht,
worin die Debriding-Formulierung topisch auf eine Wundstelle eines Patienten mit bis zu zehn Anwendungen während eines Zeitraums von bis zu vier Wochen aufgetragen wird, und
wobei die Debriding-Formulierung für mindestens vier Stunden pro Anwendung in Kontakt mit der Wundstelle gehalten wird.

2. Debriding-Formulierung zur Verwendung nach Anspruch 1,
worin die Wunde eine chronische Wunde ist, vorzugsweise worin die chronische Wunde ausgewählt ist aus der Gruppe bestehend aus einem diabetischen Ulkus, einem venösen Stauungsulkus, einem arteriellen Insuffizienzulkus, einem chronischen Druckulkus, einer chronischen postoperativen Wunde und einer chronischen posttraumatischen Wunde.

3. Debriding-Formulierung zur Verwendung nach einem der Ansprüche 1 und 2,
wobei die Debriding-Formulierung in einem Schema von bis zu 10 Anwendungen aufgetragen wird und wobei die Debriding-Formulierung für einen Zeitraum in Kontakt mit der Wundstelle gehalten wird, der ausgewählt ist aus der Gruppe bestehend aus 24 Stunden pro Anwendung, 48 Stunden pro Anwendung und 72 Stunden pro Anwendung.

4. Debriding-Formulierung zur Verwendung nach Anspruch 3,
wobei die Behandlung einmal, zweimal oder nach Bedarf wiederholt wird, bis die Wunde vollständig debridiert ist.

5. Debriding-Formulierung zur Verwendung nach einem der Ansprüche 1 bis 4,
worin die Menge an Proteinen in der Debriding-Formulierung im Bereich von 1 Gew.-% bis 5 Gew.-% des Gesamtgewichts der Debriding-Formulierung liegt; vorzugsweise worin die Menge an Proteinen in der Debriding-Formulierung bei 2 Gew.-% des Gesamtgewichts der Debriding-Formulierung liegt; oder
worin das Galaktomannan Guarkernmehl ist, das in einer Menge im Bereich von 0,25 Gew.-% bis 5 Gew.-% des Gesamtgewichts der Debriding-Formulierung vorliegt; oder
worin das Anti-Aggregationsmittel ein Oligosaccharid ist, vorzugsweise worin das Oligosaccharid ausgewählt ist aus der Gruppe bestehend aus Laktose und Saccharose, noch bevorzugter worin das Oligosaccharid Laktose ist, die in einer Menge im Bereich von 10 Gew.-% bis 25 Gew.-% des Gesamtgewichts der Debriding-Formulierung vorliegt; oder
worin das Anti-Aggregationsmittel ausgewählt ist aus Mannitol und Glukose; oder
worin das Mittel zur Einstellung des pH-Wertes Kaliumphosphat ist, das in einer Menge im Bereich von 2 Gew.-% bis 10 Gew.-% des Gesamtgewichts der Debriding-Formulierung vorhanden ist, vorzugsweise worin das Kaliumphosphat eine Kombination aus dibasischem Kaliumphosphat und monobasischem Kaliumphosphat ist, vorzugsweise worin der pH-Wert der Debriding-Formulierung im Bereich von 6,0 bis 7,0 liegt; oder
worin Wasser in einer Menge von 55 Gew.-% bis 90 Gew.-% des Gesamtgewichts der Debriding-Formulierung vorhanden ist.

6. Debriding-Formulierung zur Verwendung nach einem der Ansprüche 1 bis 5, worin die Debriding-Formulierung ferner ein Mittel umfasst, ausgewählt aus der Gruppe bestehend aus Antischaummitteln, Antioxidantien und Konservierungsmitteln.

7. Debriding-Formulierung zur Verwendung nach einem der Ansprüche 1 bis 6, worin die Debriding-Formulierung ferner einen Wirkstoff umfasst, ausgewählt aus der Gruppe bestehend aus Anästhetika, entzündungshemmenden Mitteln, Antibiotika, Antipilzmitteln, analgetischen Mitteln, Wachstumsfaktoren und heilungsfördernden Mitteln.

8. Debriding-Formulierung zur Verwendung nach einem der Ansprüche 1 bis 7, wobei die Debriding-Formulierung umfasst:
(i) die proteolytische Enzymmischung, wobei die Menge an Proteinen in der Debriding-Formulierung von 1 Gew.-% bis 5 Gew.-% des Gesamtgewichts der Debriding-Formulierung reicht;
(ii) Guarkernmehl in einer Menge im Bereich von 0,25 Gew.-% bis 5 Gew.-% des Gesamtgewichts der Debriding-Formulierung;
(iii) Laktose in einer Menge im Bereich von 10 Gew.-% bis 25 Gew.-% des Gesamtgewichts der Debriding-Formulierung;
(iv) Kaliumphosphat in einer Menge im Bereich von 2 Gew.-% bis 10 Gew.-% des Gesamtgewichts der Debriding-Formulierung; und
(v) Wasser in einer Menge, um auf 100 Gew.-% des Gesamtgewichts der Debriding-Formulierung aufzufüllen.

9. Debriding-Formulierung zur Verwendung nach Anspruch 8, worin die Debriding-Formulierung umfasst:
| Inhaltsstoff | (%) w/w der Formulierung |
|---|---|
| API | 2 |
| Guarkernmehl | 3,5 |
| Laktose | 18,05 |
| Kaliumphosphat dibasisch | 2,5 |
| Kaliumphosphat monobasisch | 0,8 |
| PEG-3350 | 2 |
| Wasser zur Injektion | 71,15 |

10. Debriding-Formulierung zur Verwendung nach Anspruch 1, wobei die Debriding-Formulierung durch die Schritte hergestellt wird:
(a) Erhalten einer Zusammensetzung in trockener oder pulverisierter Form, die umfasst:
(i) die aus Bromelain gewonnene proteolytische Enzymmischung;
(ii) das wasserlösliche Geliermittel;
(iii) das Anti-Aggregationsmittel;
(iv) das Mittel zur Einstellung des pH-Werts; und
(b) Mischen der Zusammensetzung (a) mit Wasser vor der Verwendung, um die Debriding-Formulierung zu bilden.

11. Debriding-Formulierung, welche umfasst:
(a) eine Zusammensetzung in getrockneter oder lyophilisierter Form, die umfasst:
(i) eine aus Bromelain erhaltene proteolytische Enzymmischung, die Stamm-Bromelain (EC 3.4.22.32) und Ananain (EC 3.4.22.31) umfasst;
(ii) ein wasserlösliches Geliermittel, worin das wasserlösliche Geliermittel kein vernetztes Acrylsäurepolymer ist, und worin das wasserlösliche Geliermittel ein Galaktomannan oder Glukomannan oder eine Kombination davon ist;
(iii) ein Anti-Aggregationsmittel;
(iv) ein Mittel zur Einstellung des pH-Werts; und
(b) Wasser,
wobei die Zusammensetzung (a) vor der Verwendung mit dem Wasser (b) gemischt wird, um eine Debriding-Formulierung zu bilden, die **dadurch gekennzeichnet ist, dass** sie ein homogenes Hydrogel mit einer Viskosität im Bereich von 1.800 Pa-s (1.800.000 Centipoise (cP)) bis 9.350 Pa-s (9.350.000 cP) ist, gemessen mit einem Absolutviskosimeter mit Plattengeometrie bei Raumtemperatur, und darin dass diese einen pH-Wert im Bereich von 5,4 bis 8,8 aufweist, und
worin die Menge an Proteinen in der Debriding-Formulierung von 0,5 Gew.-% bis 7 Gew.-% des Gesamtgewichts der Debriding-Formulierung reicht.

12. Debriding-Formulierung nach Anspruch 11, welche umfasst:
(a) eine Zusammensetzung in getrockneter oder lyophilisierter Form, die sich in einem ersten Kompartiment eines Behälters oder in einem ersten Behälter befindet, worin die Zusammensetzung umfasst:
(i) eine aus Bromelain erhaltene proteolytische Enzymmischung, die Stamm-Bromelain (EC 3.4.22.32) und Ananain (EC 3.4.22.31) umfasst, worin die Menge an Proteinen in der Debriding-Formulierung im Bereich von 1 Gew.-% bis 5 Gew.-% des Gesamtgewichts der Debriding-Formulierung liegt;
(ii) Guarkernmehl in einer Menge im Bereich von 0,25 Gew.-% bis 5 Gew.-% des Gesamtgewichts der Debriding-Formulierung;
(iii) Laktose in einer Menge im Bereich von 10 Gew.-% bis 25 Gew.-% des Gesamtgewichts der Debriding-Formulierung;
(iv) ein pH-Einstellmittel; und
(b) Wasser in einer Menge von 55 Gew.-% bis 90 Gew.-%, das in einem zweiten Kompartiment des Behälters oder in einem zweiten Behälter vorliegt,
wobei vor der Verwendung die Zusammensetzung (a) mit dem Wasser (b) gemischt wird, um die Debriding-Formulierung zu bilden.

13. Debriding-Formulierung nach Anspruch 12, welche umfasst:
| Inhaltsstoff | (%) w/w der Formulierung |
|---|---|
| API | 2 |
| Guarkernmehl | 3,5 |
| Laktose | 18,05 |
| Kaliumphosphat dibasisch | 2,5 |
| Kaliumphosphat monobasisch | 0,8 |
| PEG-3350 | 2 |
| Wasser zur Injektion | 71,15 |

## Revendications

1. Formulation de débridement pour une utilisation dans le débridement d'une plaie, la formulation de débridement comprenant:
(a) une composition sous une forme sèche ou en poudre comprenant:
(i) un mélange d'enzymes protéolytiques obtenu à partir de bromélaïne comprenant de la bromélaïne souche (EC 3.4.22.32), et de l'ananaïne (EC 3.4.22.31);
(ii) un agent gélifiant hydrosoluble, dans lequel l'agent gélifiant hydrosoluble est autre qu'un polymère réticulé d'acide acrylique, et dans lequel ledit agent gélifiant hydrosoluble est du galactomannane ou du glucomannane ou une combinaison de ceux-ci;
(iii) un agent antiagrégant;
(iv) un agent d'ajustement du pH; et
(b) de l'eau,
dans laquelle, avant utilisation, la composition (a) est mélangée à l'eau (b) pour former ladite formulation de débridement **caractérisée en ce qu'**elle est un hydrogel homogène ayant une viscosité dans la gamme de 1800 Pa-s (1 800 000 centipoises (cP)) à 9350 Pa-s (9 350 000 cP), mesurée par un viscosimètre absolu à géométrie de plaque à température ambiante, et ayant un pH allant de 5,4 à 8,8,
dans laquelle la quantité de protéines dans la formulation de débridement est comprise entre 0,5 % (p/p) et 7 % (p/p) du poids total de ladite formulation de débridement,
dans laquelle ladite formulation de débridement est appliquée topiquement sur le site d'une plaie d'un sujet selon un régime allant jusqu'à dix applications pendant une période allant jusqu'à quatre semaines, et
dans lequel la formulation de débridement est maintenue en contact avec le site de la plaie pendant au moins quatre heures par application.

2. Formulation de débridement à utiliser selon la revendication 1,
dans laquelle la plaie est une plaie chronique, de préférence dans laquelle la plaie chronique est choisie dans le groupe constitué par un ulcère diabétique, un ulcère de stase veineuse, un ulcère d'insuffisance artérielle, un ulcère de pression chronique, une plaie post-opératoire chronique et une plaie post-traumatique chronique.

3. Formulation de débridement à utiliser selon l'une quelconque des revendications 1 et 2,
dans laquelle la formulation de débridement est appliquée selon un schéma allant jusqu'à 10 applications, et dans laquelle ladite formulation de débridement est maintenue en contact avec le site de la plaie pendant une période de temps choisie dans le groupe constitué par 24 heures par application, 48 heures par application, et 72 heures par application.

4. Formulation de débridement à utiliser selon la revendication 3,
dans laquelle le régime est répété une fois, deux fois, ou selon les besoins jusqu'à ce que la plaie soit complètement débridée.

5. Formulation de débridement à utiliser selon l'une quelconque des revendications 1 à 4,
dans laquelle la quantité de protéines dans ladite formulation de débridement est comprise entre 1% (p/p) et 5% (p/p) du poids total de la formulation de débridement; de préférence, la quantité de protéines dans la formulation de débridement est de 2% (p/p) du poids total de la formulation de débridement; ou
dans laquelle le galactomannane est de la gomme de guar présente en une quantité allant de 0,25% (p/p) à 5% (p/p) du poids total de la formulation de débridement; ou
dans lequel l'agent antiagrégant est un oligosaccharide, de préférence l'oligosaccharide est choisi dans le groupe constitué par le lactose et le saccharose, plus préférablement l'oligosaccharide est le lactose présent en une quantité allant de 10% (p/p) à 25% (p/p) du poids total de la formulation de débridement; ou
dans laquelle l'agent antiagrégant est choisi parmi le mannitol et le glucose; ou
dans laquelle l'agent d'ajustement du pH est du phosphate de potassium présent en une quantité allant de 2% (p/p) à 10% (p/p) du poids total de la formulation de débridement, de préférence le phosphate de potassium est une combinaison de phosphate de potassium dibasique et de phosphate de potassium monobasique, plus préférablement le pH de la formulation de débridement est compris entre 6,0 et 7,0; ou
dans laquelle l'eau est présente en une quantité allant de 55% (p/p) à 90% (p/p) du poids total de la formulation de débridement.

6. Formulation de débridement à utiliser selon l'une quelconque des revendications 1 à 5, dans laquelle ladite formulation de débridement comprend en outre un agent choisi dans le groupe constitué par les agents anti-mousse, les anti-oxydants et les conservateurs.

7. Formulation de débridement à utiliser selon l'une quelconque des revendications 1 à 6, dans laquelle ladite formulation de débridement comprend en outre un agent actif choisi dans le groupe constitué par les agents anesthésiques, les agents anti-inflammatoires, les agents antibiotiques, les agents antifongiques, les agents analgésiques, les facteurs de croissance et les agents favorisant la cicatrisation.

8. Formulation de débridement à utiliser selon l'une quelconque des revendications 1 à 7, dans laquelle ladite formulation de débridement comprend:
(i) le mélange d'enzymes protéolytiques, dans lequel la quantité de protéines dans la formulation de débridement est comprise entre 1% (p/p) et 5% (p/p) du poids total de la formulation de débridement;
(ii) de la gomme de guar en une quantité allant de 0,25% (p/p) à 5% (p/p) du poids total de la formulation de débridement;
(iii) du lactose en une quantité allant de 10% (p/p) à 25% (p/p) du poids total de la formulation de débridement;
(iv) du phosphate de potassium en une quantité allant de 2% (p/p) à 10% (p/p) du poids total de la formulation de débridement; et
(v) de l'eau en une quantité permettant de compléter jusqu'à 100% (p/p) du poids total de la formulation de débridement.

9. Formulation de débridement à utiliser selon la revendication 8, dans laquelle ladite formulation de débridement comprend:
| Ingrédient | (%) p/w de la formulation |
|---|---|
| API | 2 |
| Gomme de guar | 3.5 |
| Lactose | 18.05 |
| Phosphate de potassium dibasique | 2.5 |
| Phosphate de potassium monobasique | 0.8 |
| PEG-3350 | 2 |
| Eau pour injection | 71.15 |

10. Formulation de débridement à utiliser selon la revendication 1, dans laquelle ladite formulation de débridement est préparée par les étapes suivantes:
(a) obtention d'une composition sous une forme sèche ou en poudre qui comprend:
(i) le mélange d'enzymes protéolytiques obtenu à partir de la bromélaïne;
(ii) l'agent gélifiant soluble dans l'eau;
(iii) l'agent antiagrégant;
(iv) l'agent d'ajustement du pH; et
(b) mélanger, avant utilisation, la composition (a) avec de l'eau pour former la formulation de débridement.

11. Formulation de débridement comprenant:
(a) une composition sous une forme séchée ou lyophilisée comprenant:
(i) un mélange d'enzymes protéolytiques obtenu à partir de bromélaïne comprenant de la bromélaïne souche (EC 3.4.22.32) et de l'ananaïne (EC 3.4.22.31);
(ii) un agent gélifiant hydrosoluble, dans lequel l'agent gélifiant hydrosoluble est autre qu'un polymère réticulé d'acide acrylique, et dans lequel l'agent gélifiant hydrosoluble est un galactomannane ou un glucomannane ou une combinaison de ceux-ci;
(iii) un agent antiagrégant;
(iv) un agent d'ajustement du pH; et
(b) de l'eau,
dans laquelle, avant utilisation, la composition (a) est mélangée à l'eau (b) pour former une formulation de débridement **caractérisée en ce qu'**elle est un hydrogel homogène ayant une viscosité dans la gamme de 1800 Pa-s (1 800 000 centipoises (cP)) à 9350 Pa-s (9 350 000 cP), mesurée par un viscosimètre absolu à géométrie de plaque à température ambiante, et ayant un pH allant de 5,4 à 8,8, et
dans laquelle la quantité de protéines dans la formulation de débridement est comprise entre 0,5% (p/p) et 7% (p/p) du poids total de la formulation de débridement.

12. Formulation de débridement selon la revendication 11 comprenant:
(a) une composition sous une forme séchée ou lyophilisée, présente dans un premier compartiment d'un récipient ou dans un premier récipient, la composition comprenant:
(i) un mélange d'enzymes protéolytiques obtenu à partir de bromélaïne comprenant de la bromélaïne (EC 3.4.22.32) et de l'ananaïne (EC 3.4.22.31), dans lequel la quantité de protéines dans la formulation de débridement est comprise entre 1% (p/p) et 5% (p/p) du poids total de la formulation de débridement;
(ii) de la gomme de guar en une quantité allant de 0,25% (p/p) à 5% (p/p) du poids total de la formulation de débridement;
(iii) du lactose en une quantité allant de 10% (p/p) à 25% (p/p) du poids total de la formulation de débridement;
(iv) un agent d'ajustement du pH; et
(b) de l'eau en une quantité de 55% (p/p) à 90% (p/p) présente dans un second compartiment du récipient ou dans un second récipient,
dans lequel, avant l'utilisation, la composition (a) est mélangée avec l'eau (b) pour former ladite formulation de débridement.

13. Formulation de débridement selon la revendication 12 comprenant:
| Ingrédient | (%) p/w de la formulation |
|---|---|
| API | 2 |
| Gomme de guar | 3.5 |
| Lactose | 18.05 |
| Phosphate de potassium dibasique | 2.5 |
| Phosphate de potassium monobasique | 0.8 |
| PEG-3350 | 2 |
| Eau pour injection | 71.15 |
